Europäisches Patentamt

(19) European Patent Office    (11) Publication number: **0 057 907**
Office européen des brevets                                    **B1**

(12) **EUROPEAN PATENT SPECIFICATION**

(45) Date of publication of patent specification: **30.12.86**    (51) Int. Cl.⁴: **A 61 M 1/36,** B 01 D 21/00

(21) Application number: **82100771.3**

(22) Date of filing: **03.02.82**

(54) **Apparatus for separating blood components.**

(30) Priority: **05.02.81 JP 16190/81**
**04.09.81 JP 140047/81**
**08.09.81 JP 141216/81**
**08.09.81 JP 141217/81**
**26.11.81 JP 188320/81**

(43) Date of publication of application:
**18.08.82 Bulletin 82/33**

(45) Publication of the grant of the patent:
**30.12.86 Bulletin 86/52**

(84) Designated Contracting States:
**AT BE CH DE FR GB IT LI LU NL SE**

(56) References cited:
**WO-A-80/02805**
**FR-A- 937 335**
**NL-A-7 606 791**
**US-A-3 489 145**
**US-A-3 957 197**

(73) Proprietor: **Asahi Kasei Kogyo Kabushiki Kaisha**
**2-6, Dojimahama 1-chome Kita-ku**
**Osaka-shi Osaka 530 (JP)**

(72) Inventor: **Tadaaki, Furuta**
**3669-41, Imaizumi Fuji-shi**
**Shizuoka-ken (JP)**
Inventor: **Norio, Iriguchi**
**436, Asahikaseinishi-apartment 100,**
**Kawanarijima**
**Fuji-shi Shizuoka-ken (JP)**
Inventor: **Shunsaku, Tabata**
**Asahikasei 5-ryo 100, Kawanarijima**
**Fuji-shi Shizuoka-ken (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

Courier Press, Leamington Spa, England.

**0 057 907**

**Description**

The present invention relates to an improved apparatus for separating blood components from blood by means of sedimentation action due to gravitational force.

Blood comprises a plasma component and a blood corpuscle component containing erythrocytes, leucocytes, blood platelets and the like. Recently, remarkable developments of so-called artificial organs have led to the practical use of therapeutic treatment in which blood is removed from the human body by extracorporeal circulation and subjected to a dialysis treatment or an adsorption treatment after fractionation. Such therapeutic treatment has become more and more important. It is strongly required, however, that it does not adversely affect the body. For this reason, the apparatus for the therapeutic treatment must satisfy various requirements. One of these requirements is that the apparatus should not remove the protein components contained in the patient's blood.

For example, an artificial kidney should not discharge plasma protein along with the waste liquid.

Recently, it has been learned that an abnormal increase of high molecular weight solutes such as immune complexes, cryoprecipitates, aggregates of immuno-globulin and nucleic acids in blood strongly influences the occurrence and condition of autoimmune diseases. Therefore, a plasma exchange therapy is carried out to remove said high molecular weight solutes.

Furthermore, in the field of blood transfusion, hospitals have not been transfusing the collected whole blood, but have been frequently separating the collected blood into its components in order to use the specific blood component required by the patient. One method of doing this is to allow the blood to stand, wherein the blood corpuscle component gradually settles to the bottom and separates from the plasma. However, this gravitational method of separation is very slow. For this reason, hospitals have heretofore widely used centrifugal separation for separating blood components rather than gravitational separation. Centrifugal separation, however, is disadvantageous in that it requires an expensive centrifugal separator, which in turn requires high rotational power and safety devices.

Accordingly, the objects of the present invention are to eliminate the above-mentioned disadvantages of the prior art and to provide an apparatus for effectively and efficiently separating blood components, without using artificial centrifugal force, by means of sedimentation action due to gravitational force.

Other objects and advantages of the present invention will be apparent from the description set forth hereinbelow.

In accordance with the present invention, there is provided an apparatus for separating blood components by means of sedimentation action due to gravitational force, comprising:

a first empty space provided with an opening for a feed line for blood;

a component separation portion connected with said empty space, the inside of the separation portion forming at least one blood flow channel, which is either tubular and has an inner empty space of a sectional area of not more than 3 cm$^2$, or has planar upper and lower surfaces including an empty space having a thickness of not more than 20 mm; and

a second empty space connected to said component separation portion and provided with at least two openings for discharge lines for the separated blood components.

An apparatus for separating impurities from liquid media which comprises parallel perforated plates has been known from NL—A—7 606 791. A device of this type could however not be used for separating blood components according to the present invention, since in the known apparatus the plates are provided with perforations and thus the flow channels are interconnected, while according to the present invention the walls of the blood flow channels are not perforated.

The present invention will be better understood from the description set forth below with reference to the accompanying drawings, in which:

Figure 1 is a perspective view illustrating one embodiment of the blood component separation apparatus of the present invention having a blood flow channel with planar upper and lower surfaces, in which the front is cut to make inside thereof clearly understood and in which blood is shown in the separated condition.

Figure 2 is a perspective view illustrating one embodiment of the blood component separation apparatus of the present invention having a plurality of long rectangular blood flow channels, in which the front is cut so that the inside thereof is clearly understood;

Figure 3 is a front cross-sectional view of the apparatus shown in Figure 2 taken along the line A—A' in Figure 2 in which one embodiment of the long rectangular type blood flow channel is shown.

Figure 4 is a cross-sectional view illustrating another embodiment of the long rectangular type blood flow channel;

Figure 5 is a schematic view illustrating one embodiment of the blood component separation apparatus having a plurality of the tubular type blood channels;

Figure 6 is a schematic view illustrating one embodiment of the blood component separation system in which the apparatus of Figure 1 is used;

Figure 7 is a schematic view illustrating one embodiment of the blood component separation apparatus having a plurality of the blood flow channels with planar upper and lower surfaces;

Figure 8 is a schematic view illustrating one embodiment of the blood component separation system using the apparatus shown in Figure 7;

2

Figure 9 is a perspective view illustrating another embodiment of the blood component separation apparatus according to the present invention in which the front is cut so that the inside thereof is clearly understood;

Figure 10 is a schematic view illustrating a further embodiment of the blood component separation apparatus according to the present invention, in which a plurality of blood flow channels having planar upper and lower surfaces are contained;

Figure 11 is a schematic view illustrating one embodiment of the blood component separation system in which the apparatus shown in Figure 9 is used;

Figure 12 is a perspective view illustrating a further embodiment of the blood component separation apparatus according to the present invention in which the front is cut so that the inside thereof is clearly understood; and

Figure 13 is a schematic view illustrating one embodiment of the blood component separation system for an experimental practice of the present invention.

Surprisingly, the present inventors have found that cohesive masses of erythrocytes in the form of linked coins, caused by the adhesion between the erythrocytes after passing the blood through a narrow space, do not tend to dissociate, thereby facilitating the sedimentation. Thus, blood can be efficiently and continuously separated into its components, without applying centrifugal force.

By means of the apparatus of the present invention, the desired blood component or components can be efficiently separated from the blood by just passing the blood through a blood flow channel. The separation rate in the apparatus of this invention is by several times larger, than that of the conventional simple settling method. Furthermore, it is advantageous in that the apparatus of the present invention can be used to carry out not a batch method but a continuous separation method.

The reason why the desired blood component or components can be efficiently separated from the blood according to the present invention is not clearly understood, but it would seem as follows. Although blood is a viscous liquid, the factor substantially determining the viscosity is the ratio of volume of erythrocytes to the whole blood, i.e. the hematocrit value. The hematocrit value of normal human blood is 30 through 45% and the viscosity of the normal human blood is 3 through 4 times that of water. When the hematocrit value reaches 60 through 70%, the viscosity of the blood becomes as high as 6 through 9 times that of water. On the other hand, the viscosity of the plasma component in the blood is only 1.5 through 2.0 times that of water, although it depends upon the amount and types of proteins contained in the blood. According to the observation, if blood is once slightly separated into a supernatant layer and a sedimentation layer, when flowing, by erythrocyte sedimentation and other factors, the viscosity of the sedimentation layer is increased. The higher viscosity of the sedimentation layer then creates greater resistance in the sedimentation layer against the external force causing the blood flow. Contrary to this, the lower viscosity of supernatant layer causes less resistance against said external force. On the other hand, the viscosity of blood is largely affected by the linear velocity of the blood flow. That is, a decrease of the linear velocity rapidly increases the viscosity of the blood. It appears that this phenomenon is caused by the function of cohesion, especially the adhesion between erythrocyte particles. Actual microscopic observation showed that the erythrocytes tended to form cohesive masses. These cohesive masses are frequently in the form of linked coins. This phenomenon seems to be another factor increasing the resistance to the flow. Furthermore, it is believed that the filled state of the blood flow channel during the blood flow facilitates the formation of the cohesive masses of the erythrocytes and their concentration, because, in addition to the compressing effect of the erythrocyte sedimentation (i.e. compressing effect due to downward gravitational force), the sedimentation layer is compressed by the flow (i.e. compressing effect due to lateral external force). Still furthermore, the cohesive masses of the erythrocytes in the form of linked coins, caused by the adhesion between erythrocytes, are not readily dissociated, for example, in the course of gentle mixing caused by, for example, the change of the size of the blood flow channel, and, therefore, are rapidly sedimented. It is believed that these phenomena of the blood under a flowing condition influences the function of the blood component separation apparatus of the present invention. That is to say, by means of the present apparatus, blood components can be efficiently separated without any moving parts and by just passing blood through a blood flow channel enclosing a narrow space. It has been made possible therefore to separate whole blood or a liquid containing, as a main constituent, whole blood and, as a minor constituent, a special blood component, anticoagulant or the like.

The component separation portion according to the present invention can be composed of one or a plurality of blood flow channels having planar upper and lower surfaces or one or a plurality of tubular blood flow channels. Of these flow channels, the former flow channel, for example, a long rectangular form, having a large area is desirable, since a large amount of blood can be treated by even one such flow channel.

Blood flow channels enclosing a thin flat empty space can be desirably used in the present invention, since the downward force due to erythrocyte sedimentation (i.e. gravitational force) and lateral external force due to the flow of the blood are effectively transferred to the erythrocytes and the formation of the cohesive masses is facilitated. The thickness of the blood flow channel having planar upper and lower surfaces is 20 mm or less, preferably, 0.2 through 10 mm and more preferably 0.5 through 5 mm.

Blood flow channels in the form of a tube having a small sectional area are desirably used in the present invention, since the interaction between the erythrocytes is strengthened. The sectional area of the

3

# 0 057 907

tubular flow channel is 3 cm² or less, preferably 0.0003 through 1 cm² and more preferably 0.001 through 0.5 cm².

In the case where the thickness of the flow channel having planar upper and lower surfaces is less than 0.2 mm or the sectional area of the tubular flow channel is less than 0.0003 cm² the efficiency of the separation of the plasma and the blood corpuscles tends to decrease under practical linear velocity conditions. It is believed that the above-mentioned reduction in separation efficiency is caused by a phenomenon similar to the so-called Fahraeus-Lindqvist effect. (That is, when blood flows in capillaries, blood corpuscles do not randomly flow, but are forced to flow in an oriented condition, whereby the viscosity is abnormally decreased).

The ratio of the length L (cm²) of the flow channel to the inlet sectional area S (cm²) of the flow channel (i.e. L/S) in the blood flow channel of the present invention is desirably 10 or more to strengthen the interaction of the erythrocytes.

In the use of the apparatus of the invention the linear velocity of the blood flow is desirably 0.5 through 200 mm/min more desirably 1 through 100 mm/min and most desirably 2 through 50 mm/min. In the case where the linear velocity of the blood flow is larger than 200 mm/min effective and efficient blood component separation becomes difficult, as it is likely to cause a turbulent flow and remarkably weaken the cohesion between the erythrocyte particles. On the other hand, in the case where the linear velocity of the blood flow is smaller than 0.5 mm/min the capacity for treatment of the blood is reduced. Although the length of the blood flow channel can be shortened, in such case, in order to increase the capacity for treatment of the blood, the width of the blood flow channel would have to be increased. Such an increase, however, would likely cause channelling of the blood. The width of the blood flow channel is desirably 50 through 500 mm more desirably 100 through 300 mm.

The temperature of the blood flow is desirably 35 through 42°C more desirably 37 through 40°C. The separation efficiency becomes large, as the temperature of the blood to be separated becomes high. However, in the case where the temperature of the blood becomes too high, the hemolysis of the erythrocytes and the denaturation of enzymes might be caused.

The flow rate of the blood is such that the residence time of the blood in the blood flow channel is desirably 2 through 20 min more desirably 4 through 15 min, although it may be varied depending upon, for example, the shape or the volume of the blood flow channel. A too short residence time causes insufficient blood separation due to a too large flow rate of the blood. On the other hand, the too long residence time results in the undesirably small volume of the separated blood component or components obtained by the apparatus due to the too small flow rate of the blood. As a general trend, the narrower the thickness of the blood flow channel or the smaller the sectional area of the blood flow channel, the greater is the amount of the separated blood component or components obtainable in a short residence time.

The term "residence time T (min)" used herein is defined by the following equation:

$$T = \Sigma V / Q$$

Wherein
$\Sigma V$: total volume of blood flow channel (ml)
$Q$: flow rate of blood (ml/min)

The blood flow channel is desirably mounted in such a manner that the blood to be separated flows in the blood flow channel in an upward direction at an angle more than the substantial horizontal plane but less than approximately 45°. The term "substantial horizontal plane" includes an inclination within approximately ±10° from the horizontal plane. A downward direction of flow is undesirable as an air layer is likely to remain in the blood flow channel. Contact of the blood with an air layer tends to cause coagulation of the blood during the continuous flow of the blood and the blood flow should be carefully deaerated to remove the air layer. Too great an upward angle of flow is also undesirable, since the higher the angle, the lower the separation efficiency. For easy deaeration and effective space utilization, however, one can set the angle to as much as approximately 45° in practical use, though an angle of 20° or less would be more desirable. Of course, it should be noted that not necessarily the whole blood flow channel has to be upwardly inclined. The inclusion of any descending portion in the channel, however, is not desirable due to the fact that air retaining portions may be formed.

The previous addition of a water-soluble polymer to the blood to be separated promotes the very effective cohesion of the erythrocytes and, therefore, the supernatant layer can be rapidly collected. The water-soluble polymers usable for this purpose include, for example, gelatin and the derivatives thereof, polysaccharides such as dextran, insulin and the derivatives thereof and water-soluble polyvinyl compounds such as polyvinyl alcohol, polyvinyl pyrrolidone and the derivatives thereof. Water-soluble polymers having a molecular weight of more than 1000 promote the cohesion of the erythrocytes. However, the use of water-soluble polymers having a too large molecular weight is not recommendable due to the facts that they have antigenic properties and also that they adversely affect the kidney. Accordingly, water-soluble polymers having a molecular weight of less than 100,000 but more than 1000, more preferably less than 50,000 but more than 1000 can be suitably used.

The water-soluble polymers are desirably used in the form of an aqueous solution having the approximately same osmotic pressure as the physiological osmotic pressure, in order to continuously separate the blood components. The desired concentration range of the water-soluble polymers in water is within the range of from 1 to 10% by weight. The amount of the aqueous water-soluble polymer solution added to the blood is desirably within the range of from 10 to 25% by volume.

The separation portion having a plurality of blood flow channels having planar upper and lower surfaces can be readily made by piling up multiple flat plates at a constant distance. For instance, spacers can be inserted between the flat plates, to ensure a fairly constant thickness of the blood flow channel. Plates with protrusions having an appropriate height can also be laminated to further divide the spaces into smaller sections.

The tubular blood flow channels can be in the form of any shape, for example, a circular column, an elliptical column or a hexagonal column. Tubes in the form of circular columns and hexagonal columns can be suitably used in the present invention because they can be readily bundled together and adhered to one another. By the use of these tubular materials, the apparatus for separating blood components can be readily manufactured.

In the apparatus according to the present invention, the second empty space having the openings for the discharge lines of the separated blood components desirably has approximately the same or a smaller volume than the total volume of one or a plurality of the blood flow channels in the component separation portion. On the other hand, since the first empty space having an opening for the feed line of the blood is provided for distributing the blood to be fed into one or a plurality of the blood flow channels as uniformly as possible, it may have any shape as long as said purpose is attained. However, the volume of the first empty space is desirably as small as possible.

The blood flow channels are desirably provided with surfaces which are substantially flat. The term "substantially flat" does not necessarily mean a microscopic smoothness. However, relatively rough materials can be used for the flow surfaces to provide some additional resistance against the blood flow. Furthermore, irregular portions can be provided on the surfaces of the blood flow channels. Still further, the surface area of the flow surface of the blood flow channel can be enlarged by using a corrugated plate having corrugations parallel to the direction of the blood flow.

From a practical point of view, the volume of the apparatus according to the present invention is at least 50 ml. Furthermore, when the apparatus of the present invention is used for the purpose of external circulation of human blood by connecting the apparatus to a human blood vessel, or when separating a one-man portion of collected blood contained in a blood bag the volume of the apparatus is desirably 500 ml or less.

When blood is separated by the apparatus according to the present invention, the blood is desirably passed through the second empty space having the openings for the discharge lines of the separated blood components such that the residence time of the blood therein is 2 min or more.

The preferred embodiments of the present invention will now be illustrated in detail in connection with the accompanying drawings.

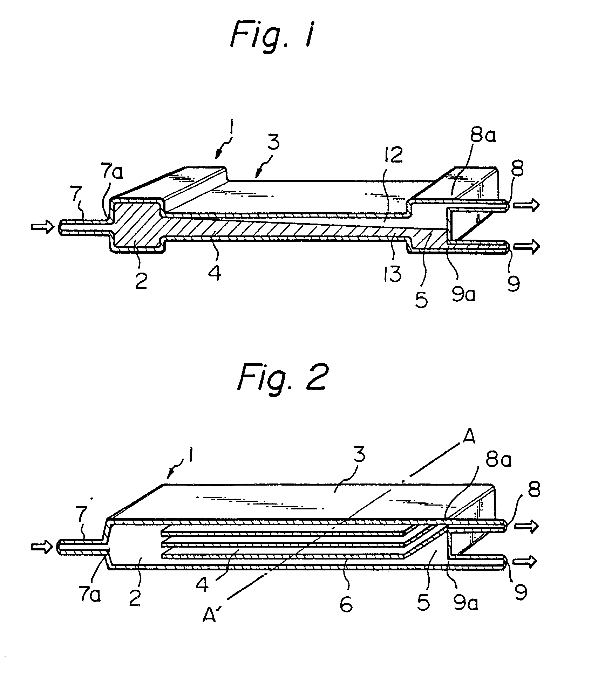

In Figure 1, a sealed vessel 1 contains, as a component separation portion, one blood flow channel 4 having a substantially flat bottom plate. The bottom plate is maintained in such a manner that the blood to be separated is passed through the blood flow channel 4 in a substantially horizontal direction or upward direction. The blood, usually containing an anticoagulant, is continuously fed through a feed line 7 via an opening 7a to an empty space (A) 2. The blood thus fed is gradually separated, by the blood cohesion, along the blood flow channel into a supernatant layer 12 comprising platelet rich plasma and a sedimentation layer 13 comprising blood corpuscles containing erythrocytes and leucocytes. The sedimentation layer 13 is sometimes further separated, whereby a leucocyte layer floating on an erythrocyte layer is formed, as an intermediate layer. The separated components are continuously discharged from an empty space (B) 5 via openings 8a and 9a, which are located in each component layer, through a discharge line 8 and a discharge line 9. In this case, the openings 8a and 9a are provided at the uppermost portion and the lowermost portion of the blood component streams at the end of the downstream side of the empty space (B) 5, respectively. Thus, the platelet rich plasma is discharged through the discharge line 8 and the blood corpuscles are discharged through the discharge line 9.

For better effect, one can previously add plasma to the blood to be introduced through the feed line 7. This previous addition of plasma to the blood increases the amount of the platelet rich plasma discharged through the discharge line 8. Experiments have confirmed, however, that even when said previously added plasma contains substantially no platelets, the amount of platelets contained per unit amount of the platelet rich plasma discharged through the discharge line 8 decreases at a smaller rate than said increase in the amount of platelet rich plasma. As a result, the amount of the separated and discharged blood platelets per unit time is increased.

Furthermore, as mentioned hereinabove, the erythrocyte sedimentation effect is essential to the present invention. However, the erythrocyte sedimentation effect is sometimes small for certain types of blood to be separated, especially when the blood is in the state of polycythemia. In this case, the inclusion of plasma in the blood to be fed increases the effect of the present invention.

In addition, in the case of very anemic blood having a hematocrit value of 20% or less, concentrated erythrocytes can be previously added to the blood to be fed, or a portion of the blood corpuscles discharged from the discharge line 9 can be previously added to the blood to be fed, to effectively increase the probability of erythrocyte cohesion.

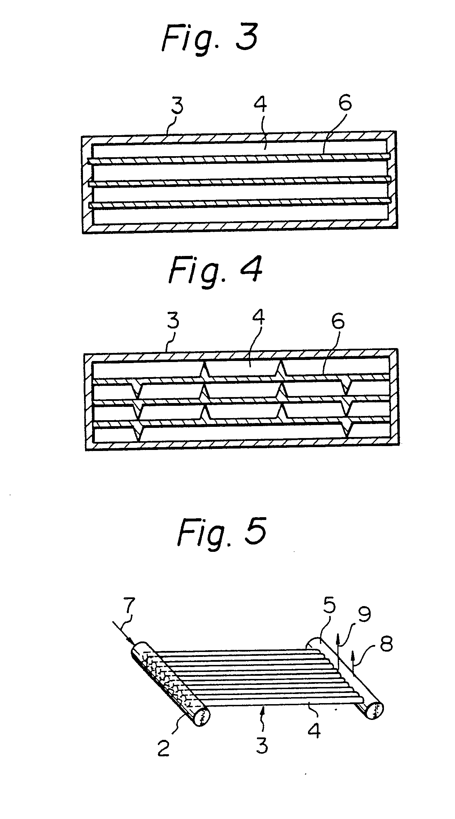

The embodiments of the apparatuses of the present invention shown in Figures 2 and 5 can also be used in the same manner as that of Figure 1. That is to say, blood to be separated is fed through the opening 7a to the empty portion (A) 2 and, then, transferred to a plurality of the blood flow channels 4 in the component separation portion 3. Thus, the blood cohesion is promoted in the blood flow channels, and the supernatant layer comprising platelet rich plasma and the sedimentation layer comprising blood corpuscles are discharged from the empty space (B) 5 via the discharge lines 8 and 9, respectively.

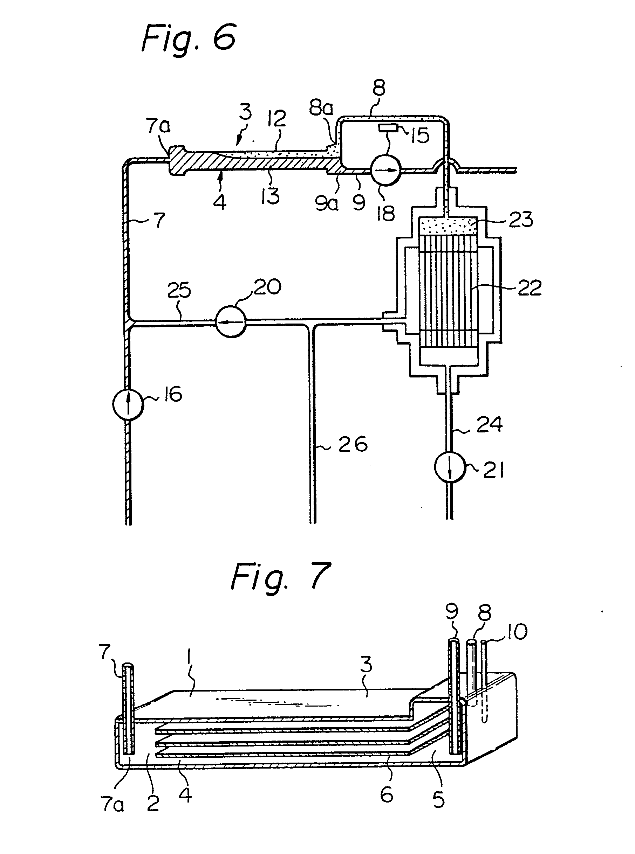

In Fig. 6 blood to be separated is fed to a blood flow channel 4 in a separation portion 3 through a feed line 7 via an opening 7a by means of a pump 16. The blood fed to the blood flow channel 4 is separated into a supernatant layer 12 comprising platelet rich plasma and a sedimentation layer 13 comprising blood corpuscles. The supernatant layer 12 is discharged from a discharge line 8 through an opening 8a, and the sedimentation layer 13 is discharged from a discharge line 9 through an opening 9a.

In order to detect whether or not components of the sedimentation layer are included in the discharged supernatant layer, a detector 15 is installed in the discharge line 8. A pump 18 connected to the detector 15 is installed in the discharge line 9. That is, when the detector 15 detects contamination of the supernatant layer by components of the sedimentation layer, it actuates the pump 18 to initiate the discharge of the sedimentation or to increase the discharge flow rate of the sedimentation layer. As a result, the separation boundary surface between the supernatant layer and the sedimentation layer is moved downwardly. Thereafter, when the detector 15 no longer detects the above-mentioned contamination, the pump 18 is actuated to stop the discharge or to decrease the discharge flow rate. Thus, the separation boundary surface in the blood flow channel is controlled to a predetermined level. As a detector, an optical type detector can be suitably used due to the fact that an optical type detector does not adversely affect the separated components. Generally speaking, an optical type detector is suitable for use in the detection of the contamination of plasma by blood corpuscles rather than detection of the contamination of blood corpuscles by plasma.

The platelet rich plasma discharged through the discharge line 8 is introduced into a filter chamber 23 provided with porous membrane filter 22, wherein platelet poor plasma is filtered and the plasma containing concentrated blood platelet is discharged through a discharge line 24 by means of a pump 21. The filtered platelet poor plasma is discharged through a discharge line 26, and a portion thereof is added to the blood to be fed to the blood flow channel, through the feed line 7 via a tube 25, by means of a pump 20, for the above-mentioned technical reasons. Porous membrane filters suitably used in the present invention are those which have an approximately uniform pore size of a maximum 2 microns or less. In the case where the maximum pore size of the membrane filter is less than 0.1 micron, not only the blood platelets contained in the platelet rich plasma are concentrated, but also the protein component tends to be concentrated and, further, the concentration rate is undesirably reduced. On the other hand, in the case where the maximum pore size of the membrane filter is greater than 2 microns, blood platelets having a relatively small size tend to be filtered. The desirable pore size is 0.1 through 1 microns, more desirably 0.1 through 0.5 micron. A maximum pore size greater than 1 micron tends to result in adherence and build-up of platelets on the surface of the porous membrane filter, thereby clogging the membrane filter. However, in the case where the maximum pore size of the membrane filter is 1 µm or less, not only the filtration of the blood platelet through the membrane filter is stopped, but also the built-up or clogging of the blood platelets on the surface of the membrane filter is effectively prevented. As a result, the concentrated blood platelets can be readily obtained.

A porous membrane filter made of polyethylene, polypropylene, cellulose acetate, polycarbonate, polyvinylidene fluoride and the like is desirably used in the present invention. For effective filtration, the pressure difference across the membrane filter is practically 133,3 through 66614 Pa, desirably 666,14 through 13332 Pa. Although the porous membrane can be a flat membrane, a membrane filter in the form of hollow fibers is desirably used in the present invention due to its relatively compact construction.

The flow rate of the platelet rich plasma to be fed to the filter chamber can be widely determined depending upon, for example, the opening space ratio of the membrane filter, the filtering pressure and the area of the membrane filter. For instance, a flow rate of 4 through 40 ml/min can be practically used. The flow rate of the platelet concentrated plasma discharged through the discharge line 24 can be practically 1/20 through 1/2 of the flow rate of the platelet rich plasma introduced into the filter chamber 23. In the case where the flow rate is less than 1/20, the blood platelets tend to adhere onto the surface of the porous member filter, whereby the membrane filter is clogged and the blood platelets build up on the surface of the membrane filter. Contrary to this, in the case where the flow rate ratio is greater than 1/2, the desired concentration of the plasma can not be achieved.

Figure 7 illustrates one embodiment of the blood component separation apparatus of Figure 1. This apparatus contains a plurality of blood flow channels 4 and discharge lines 8, 9, and 10 for discharging the supernatant layer, sedimentation layer, and intermediate layer from the empty space (B) 5.

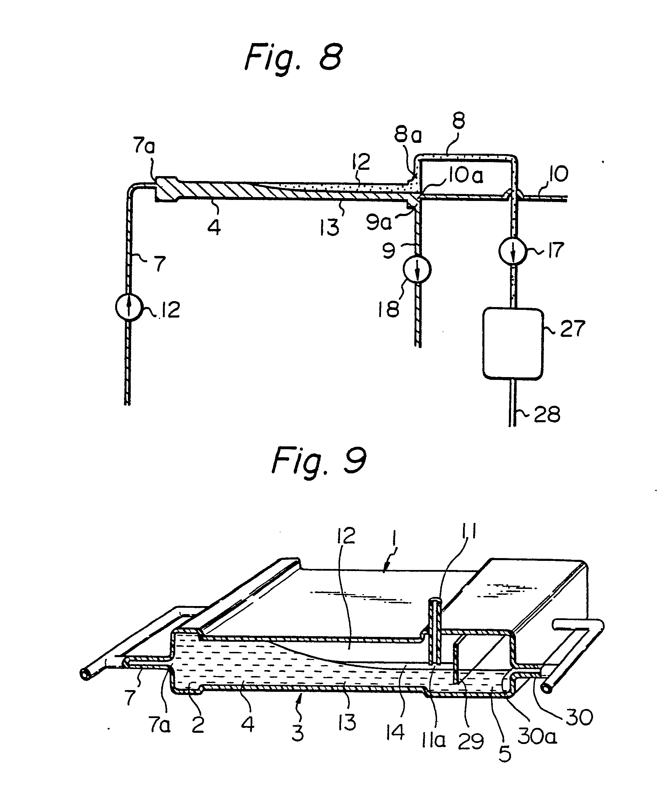

In Figure 8 illustrating one embodiment of the blood component separation system according to the present invention, the supernatant layer 12 separated in the blood flow channel 4 is discharged from a discharge line 8 and introduced into a column 27 by means of a pump 17. On the other hand, the sedimentation layer 13 separated in the blood flow channel 4 is discharged through a discharge line 9 by means of a pump 18 to the outside of the system. In the case where the separation boundary surface of the supernatant layer 12 and the sedimentation layer 13 moves up from the level of the opening 10a, the component of the sedimentation layer 13 can be discharged through the opening 10a. On the other hand, when said boundary surface moves down from the level of the opening 10a, the component of the supernatant layer 12 can be discharged through the opening 10a. Thus, the level of said separation boundary surface can be controlled to a desired level and the contamination of the platelet rich plasma discharged through the discharge line 8 by the component of the sedimentation layer can be eliminated. When the separation further proceeds, an intermediate layer of leucocytes sometimes forms and floats on the erythrocyte layer. This intermediate layer of leucocytes can also be discharged through the discharge line 10 to the outside of the system.

The column 27 is packed with an adsorbing substance, for example, acrylic fibers. The blood platelets floating in the platelet rich plasma and the leucocytes contained therein are adsorbed by the adsorbing substance and removed from the plasma and, therefore, platelet poor plasma can be discharged through a discharge line 28. As the adsorbing substances, in addition to the acrylic fibers, various natural or synthetic fibers such as polyester, polyamide, rayon, cotton, and silk can be used in the present invention. Although there is no critical limitation on the denier of these fibers, practically fibers having a denier of 0.1 through 5 can be used. Furthermore, the column 27 can be practically packed with these fibers at a packing density of 0.05 through 0.5 g/ml and the volume of the column 27 can be practically 10 through 100 ml, in the case where 500 ml of the platelet rich plasma is treated.

A column in which activated carbon, an ion exchange resin, alumina or the like is packed or an affinity column in which an antigen-antibody reaction is carried out can also be used for column 27 in the present invention, whereby a soluble toxic substance contained in the plasma can be removed.

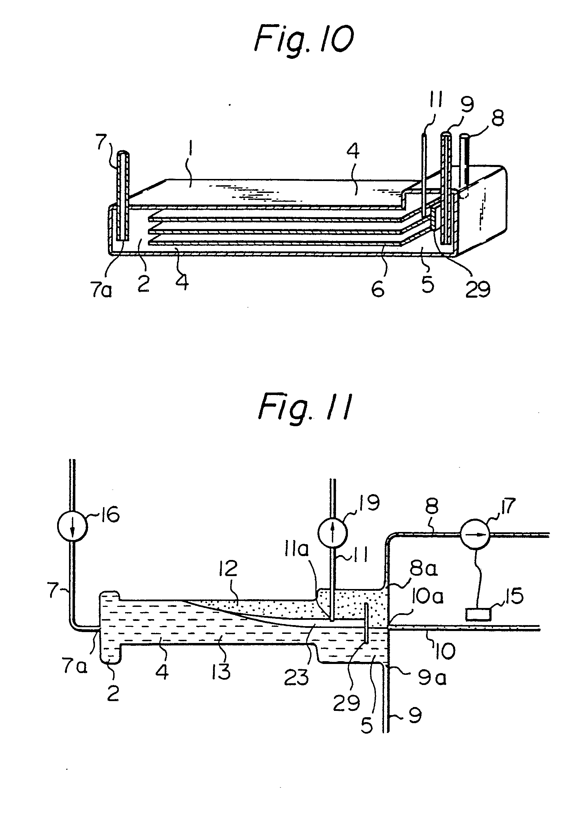

In Figure 9, a weir 29 is mounted at the downstream side of the blood flow channel 4 of the vessel 1 but at the upstream side of the empty space (B) 5 in such a manner that only the flow of the intermediate layer containing a large amount of leucocytes can be blocked. In order to discharge the blocked intermediate layer 14, an opening 11a is provided at the upstream side of the weir 29. An opening 30a is provided at the downstream side of the weir 29 in such a manner that it is somewhat lower in level than that of the opening for a discharge line 11. The supernatant layer 12 flows over a weir 29, and is discharged with the sedimentation layer 13 through a discharge line 30. However, in the case where the separation boundary surface between the supernatant layer 12 and the sedimentation layer 13 moves up from the level of the opening 30a, the component contained in the supernatant layer is discharged through the opening 30a. On the other hand, when the separation boundary surface moves downward from the level of the opening 30a, the component contained in the sedimentation layer is discharged through the opening 30a. This allows the level of the separation boundary surface to be controlled, whereby the level of the intermediate layer 14 which is located approximately on the separation boundary surface is controlled. It should be noted that a further discharge line for discharging the supernatant layer 12 comprising platelet rich plasma can be provided as shown in Figure 10. Furthermore, a still further discharge line 11 for discharging the intermediate layer can be provided, in addition to the above-mentioned two further discharge lines 8 and 9 in the sealed vessel 1, instead of the discharge line 30 of Figure 9. In Figure 9, the component of the sedimentation layer 13 is often included in the intermediate layer discharged through the discharge line 11. Accordingly, the intermediate layer 14 discharged through the discharge line 11 is introduced into another apparatus of the present invention, wherein the component of the sedimentation layer 13 can be sedimented and removed.

In Figure 11, a pump 19 is mounted on the discharge line 11. An opening 10a of a discharge line 10 is provided at the downstream side of the weir 29 and at a level approximately the same as that of the opening 11a of the discharge line 11. In the discharge line 10, a detector 15 is provided for detecting the concentration of the supernatant layer 12 or sedimentation layer 13 discharged from the opening 10a. A pump 17 connected with the detector 15 is mounted in a discharge line 8. When the detector 15 detects the presence of the component of the sedimentation layer 13, it actuates the pump 17 to decrease the discharge flow rate or stops the pump 17. As a result, the separation boundary surface between the supernatant layer 12 and the sedimentation layer 13 moves downward. When the detector 15 no longer detects the above-mentioned component, it actuates the pump 17 to increase the discharge flow rate or starts the pump 17. When the weir 29 and the discharge line 11 are not mounted, the intermediate layer 23 is discharged through the discharge line 10.

The sealed vessel used in the present invention should be made of rigid materials which will not deform by inside pressure. Furthermore, the sealed vessel should be made of nontoxic and antithrombosis materials. However, it is also possible to make the sealed vessel from nonrigid thin materials being supported by a rigid material. The materials which can be used in the manufacture of the sealed vessel include rigid materials such as polycarbonate resin, rigid polyvinyl chloride resin, acrylic resin, and aluminum and nonrigid materials such as silicone resin, nonrigid, polyvinyl chloride resin, and polyethylene resin.

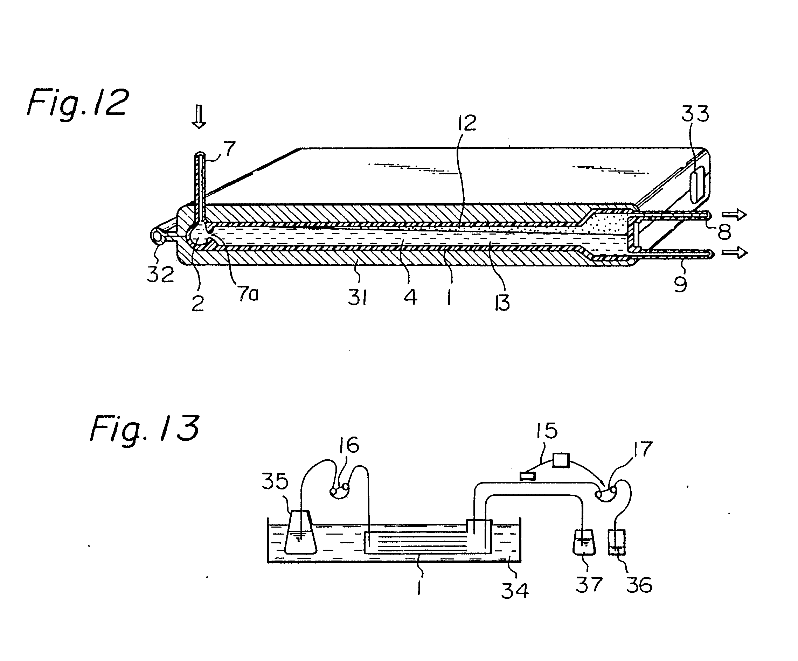

Figure 12 is a perspective view illustrating a practical embodiment of the blood component separation apparatus of the present invention, in which a nonrigid sealed vessel is supported by a stiff material. That is, the vessel 1 in Figure 12 is made of silicone resin and, in order not to deform by inside pressure, the silicon resin vessel is substantially supported in a shell 31 made of aluminum. The shell 31 has a high heat transfer coefficient and, therefore, is suitable for heating the blood contained in the vessel 1. Of course, in order to heat the shell 31, an appropriate heater can be embedded therewithin. The shell 31 can be opened and closed by means of a hinge 32 and can be locked by a means 33.

Since blood component separation vessels are usually made disposable from a sanitary point of view, the embodiment as shown in Figure 12 is advantageous.

As mentioned hereinabove, by means of the apparatus of the present invention, blood components can be separated by using a very simple step and means, without applying centrifugal force to the blood, whereby erythrocyte concentrated blood and platelet rich plasma, erythrocyte concentrated blood, leucocyte concentrated blood, and platelet rich plasma can be collected. Furthermore, concentrated platelet liquid and platelet poor plasma can be obtained from the platelet rich plasma obtained above.

By using this apparatus, substances toxic to organisms can also be removed from platelet rich plasma, whereby the desired therapeutic purposes can be attained. Since the apparatus according to the present invention can be readily used even in, for example, a small room, it is extremely advantageous for plasma exchange therapy and plasma purification therapy for patients having a diffuse collagen disease such as rheumatoid arthritis showing the sthenia of erythrocyte sedimentation and cancer patients.

The invention has been described in detail with particular reference to certain preferred embodiments thereof, but it will be understood that variations and modifications can be effected by those skilled in the art within the scope of the invention as defined by the claims.

The present invention will now be further illustrated by, but is by no means limited to, the following examples.

Example 1

Blood component separation apparatuses as shown in Figure 1 were made using acrylic resin. The dimensions are shown in Table 1 below. Blood component separation experiments were carried out using these apparatuses in an experimental system as shown in Figure 13.

The blood component separation experiments were carried out as follows:

The apparatus 1 and a fresh blood storage vessel 35 were warmed in a constant temperature bath 34. Porcine blood, having an erythrocyte sedimentation rate of 56 mm (1 hr) at 37°C and to which heparin was added to prevent the coagulation, was warmed to a temperature of 37°C in the vessel 35. The blood was fed to the blood flow channel at a flow rate of 10 ml/min by means of the pump 16 for the separation of the blood components. The concentrated erythrocytes forming the sedimentation layer were discharged from the apparatus, and collected in an erythrocyte concentrated blood receiving vessel 37. The platelet rich plasma separated in the apparatus 1 was pumped up by the pump 17 and collected in the platelet rich plasma receiving vessel 36. When an erythrocyte detector 15 detected an increase in the erythrocyte concentration of the platelet rich plasma, the pump 17 was stopped.

The flow rate and the separation efficiency (amount of plasma/amount of whole blood) (%) of the platelet rich plasma thus obtained are shown in Table 1 below.

TABLE 1

| Exp. No. | Blood flow channel vol. (cm³) | Thickness (mm) | Linear velocity of blood (cm/min) | Residence time (min) | Flow rate of platelet rich plasma (ml/min) | Separation efficiency (%) |
|---|---|---|---|---|---|---|
| 1 | 52 | 1 | 5.0 | 5.2 | 3.8 | 38 |
| 2 | 52 | 2 | 5.0 | 5.2 | 3.2 | 32 |
| 3 | 65 | 5 | 2.0 | 6.5 | 2.3 | 23 |
| 4 | 195 | 15 | 0.67 | 19.5 | 1.6 | 16 |

Example 2

Blood component separation apparatuses having the same thickness as that of No. 2 of Example 1 were manufactured. Blood separation experiments were carried out using these apparatuses at the same flow rate of blood as described in Experiment No. 2 of Example 1. The erythrocyte sedimentation rate of the porcine blood used was 46 mm at 37°C. The volumes of a blood flow channel of the apparatuses and the results of the separation experiments are shown in Table 2 below.

8

TABLE 2

| Exp. No. | Blood flow channel vol. (cm³) | Residence time (min) | Flow rate of platelet rich plasma (ml/min) | Separation efficiency (%) |
|---|---|---|---|---|
| 1 | 40 | 4.0 | 1.3 | 13 |
| 2 | 80 | 8.0 | 2.7 | 27 |
| 3 | 120 | 12.0 | 3.5 | 35 |

Example 3

A blood component separation apparatus having the same dimensions as that of experiment No. 2 of Example 1 was manufactured. Blood component separation experiments were carried out in the same manner as described in Example 1 except that the flow rate of the blood was changed and the erythrocyte sedimentation rate of the porcine blood used was 49 mm at 37°C. The results obtained from the separation experiments are shown in Table 3 below.

TABLE 3

| Exp. No. | Flow rate of blood (ml/min) | Residence time (min) | Flow rate of platelet rich plasma (ml/min) | Separation efficiency (%) |
|---|---|---|---|---|
| 1 | 12 | 4.3 | 1.8 | 15 |
| 2 | 7.5 | 6.9 | 1.9 | 25 |
| 3 | 3.5 | 14.9 | 1.3 | 37 |

Example 4

A blood component separation apparatus as shown in Figure 1 having a volume of a blood flow channel of 300 cm³ and a thickness of the blood flow channel of 2 mm was manufactured using acrylic resin. Blood separation experiments were carried out in the same manner as described in Example 1, except that the flow rates of the blood were changed and the erythrocyte sedimentation rate of the porcine blood was 81 mm at 37°C. The results obtained from the separation experiments are shown in Table 4 below. As Comparative Examples, the separation results obtained from comparative experiments in which blood was allowed to stand in the same vessel are also shown in Table 4 below. In Table 4, experiment Nos. 1 through 5 are working examples and experiments Nos. 6 through 10 are Comparative Examples.

TABLE 4

| Exp. No. | Method | Flow rate of blood (ml/min) | Residence time (min) | Flow rate of platelet rich plasma | Separation efficiency (%) |
|---|---|---|---|---|---|
| 1 | Present invention | 100 | 3 | 14.1 ml/min | 14 |
| 2 | Present invention | 60 | 5 | 19.9 ml/min | 33 |
| 3 | Present invention | 20 | 15 | 8.0 ml/min | 40 |
| 4 | Present invention | 10 | 30 | 4.6 ml/min | 46 |
| 5 | Present invention | 5 | 60 | 2.5 ml/min | 50 |
| 6 | Settling | (300) | 2 | ~0 (ml/hr) | 0 |
| 7 | Settling | (300) | 5 | 10 (ml/hr) | 0 |
| 8 | Settling | (300) | 15 | 25 (ml/hr) | 8 |
| 9 | Settling | (300) | 30 | 65 (ml/hr) | 22 |
| 10 | Settling | (300) | 60 | 80 (ml/hr) | 27 |

Example 5

A blood separation apparatus as shown in Figure 1 was manufactured using acrylic resin. The volume of the blood flow channel was 26 ml and the thickness of the blood flow was 2 mm. Porcine blood having an erythrocyte sedimentation rate of 65 mm at 37°C and to which heparin was added as an anticoagulant was fed through a blood feed line at a flow rate of 5.4 ml/min. The linear velocity of the blood flow was 27 mm/min.

While the temperature of the experimental system was kept at 37°C blood separation experiments were carried out by changing the degree of inclination of the blood flow channel. The flow rate of the platelet rich plasma discharged through the discharge line 8 and the separation efficiency are shown in Table below.

TABLE 5

| Inclination (degree) | Flow rate of plasma (ml/min) | Separation efficiency (%) |
|---|---|---|
| −12 | 0 | 0 |
| −8 | 0.43 | 8.0 |
| 0 | 1.81 | 33 |
| 11 | 1.57 | 29 |
| 15 | 1.34 | 25 |
| 21 | 1.10 | 20 |
| 29 | 0.90 | 17 |
| 45 | 0.45 | 8.3 |
| 60 | 0.09 | 1.7 |

Example 6

Blood separation apparatuses as shown in Figure 2 and Table 6 were manufactured using acrylic resin

10

for the vessel and the blood flow channel plates and using stainless steel for blood feed line and discharge lines.

Blood component separation systems as shown in Figure 13 are assembled by using the apparatuses manufactured above. Porcine blood containing heparin added for preventing the coagulation and having a temperature of 37°C was fed through a blood feed line at a flow rate of 5.2 ml/min whereby the blood components were separated. The results are shown in Table 6 below.

TABLE 6

| Exp. No. | Total volume of vessel $(cm^3)$ | Area of flow channel $(cm^2)$ | Thickness of flow channel (cm) | Number of flow channel | Volume of second empty space $(cm^3)$ | Flow rate of platelet rich plasma obtained (ml/min) |
|---|---|---|---|---|---|---|
| 1* | 75.5 | 10×13 | 2 | 2 | 13.5 | 1.6 |
| 2* | 149 | 10×13 | 2 | 5 | 7.3 | 0.8 |
| 3** | 78.5 | 10×13 | 1 | 5 | 7.3 | 0.8 |
| 4** | 78.5 | 10×11 | 1 | 5 | 13.5 | 1.3 |
| 5** | 78.5 | 10×11 | 1 | 5 | 7.3 | 0.7 |

\* Erythrocyte sedimentation rate 70 mm (1 hr. 37°C)
\*\* Erythrocyte sedimentation rate 45 mm (1 hr. 37°C)

Example 7

Blood separation apparatuses as shown in Figure 5 and Table 7 were manufactured using acrylic resin for the vessel and using stainless steel pipes for the blood feed line, the blood discharge lines and the tubular blood flow channels.

Blood component separation systems as shown in Figure 13 were assembled by using the apparatuses manufactured above. Porcine blood containing heparin added to prevent the coagulation and having a temperature of 37°C were fed through the blood feed line at a flow rate of 1.5 ml/min whereby the blood components were separated. The results are shown in Table 7 below.

TABLE 7

| Exp. No. | Total volume of vessel $(cm^3)$ | Diameter of pipe (mm) | Length of pipe (cm) | Number of pipe | Volume of Second empty space $(cm^3)$ | Flow rate of platelet rich plasma obtained (ml/min) |
|---|---|---|---|---|---|---|
| 1* | 13.7 | 2 | 12 | 20 | 3.1 | 0.42 |
| 2* | 16.2 | 2 | 12 | 40 | 0.6 | 0.15 |
| 3** | 14.1 | 1 | 10 | 100 | 3.1 | 0.54 |
| 4** | 21.0 | 5 | 15 | 5 | 3.1 | 0.31 |
| 5** | 59.4 | 15 | 30 | 1 | 3.1 | 0.16 |

\* Erythrocyte sedimentation rate 20 mm (1 hr. 37°C)
\*\* Erythrocyte sedimentation rate 45 mm (1 hr. 37°C)

Example 8

A blood separation apparatus as shown in Figure 2 was manufactured using acrylic resin. The thickness of the blood flow channel was 2 mm the volume thereof 30 $cm^3$, the volume of the empty space (B) 15 $cm^3$ and the total volume of the separation apparatus 50 $cm^3$.

The blood separation experiments were carried out by using the apparatus manufactured above. The temperature of the apparatus was maintained at 37°C. Porcine blood containing heparin added to prevent

11

the coagulation and having a temperature·of 38°C was fed through the blood feed line at a flow rate of 10 ml/min. while a water-soluble polymer is simultaneously added to the blood.

The erythrocyte sedimentation rate of the porcine blood was 43 mm at 37°C.

The following water-soluble polymers were used.

Gelatin derivatives

Gelafsin (manufactured by Toyo Jyozo Co. in Japan; polymerization product of decomposed gelatin 4% sodium chloride 0.852% and calcium 0.074 g).

Polysaccharide

Dextran 70 (dextran intraveous infusion manufactured by Otsuka Kojyo in Japan; dextrose 5%, dextran 70 (M.W. about 70,000) 6%)

Dextran 40 (low molecular dextran intraveous infusion manufactured by Otsuka Kojyo in Japan; dextrose 5%, dextran 40 (M.W. about 40,000) 10%).

The results are shown in Table 8 below.

TABLE 8

| Exp. No. | Aqueous water-soluble polymer solution | | Flow rate of platelet rich plasma (ml/min) | Separation efficiency* (%) |
|---|---|---|---|---|
| | Type | flow rate | | |
| 1 | Gelafsin | 2 ml/min | 5.7 | 37 |
| 2 | Dextran 70 | 2 ml/min | 5.1 | 31 |
| 3 | Dextran 40 | 2 ml/min | 5.2 | 32 |
| 4 | — | — | 2.7 | 27 |

$$*Separation\ efficiency = \frac{\left(\begin{array}{c}Flow\ rate\ of\ platelet \\ rich\ plasma\end{array}\right) - \left(\begin{array}{c}Flow\ rate\ of\ aqueous\ water- \\ soluble\ polymer\ solution\end{array}\right)}{(Flow\ rate\ of\ blood\ containing\ anticoagulant)}$$

## Claims

1. An apparatus for separating blood components by means of sedimentation action due to gravitational force, comprising:

a first empty space (2) provided with an opening for a feed line (7) for blood;

a component separation portion connected with said empty space, the inside of the separation portion forming at least one blood flow channel (4), which is either tubular and has an inner empty space of a sectional area of not more than 3 cm², or has planar upper and lower surfaces including an empty space having a thickness of not more than 20 mm; and

a second empty space (5) connected to said component separation portion and provided with at least two openings (8, 9) for discharge lines for the separated blood components.

2. An apparatus as claimed in Claim 1, wherein two openings for the discharge lines (8, 9) are located at two different heights in the second empty space.

3. An apparatus as claimed in Claim 1, wherein three openings for the discharge lines (8, 9, 10) are located at different heights in the second empty space.

4. An apparatus as claimed in any one of Claims 1 to 3, wherein a weir (29) is mounted at an intermediate height in the second empty space (5) in such a manner that the weir blocks a portion of the blood flow and wherein one opening (11a) for a discharge line (11) is located at an intermediate height at the upstream side of the weir (29) and at least one opening (30a) for a discharge line is located at the downstream side of the weir.

5. An apparatus as claimed in any of the Claims 1 to 4, wherein the blood flow channels (4) are inclined upwardly in the direction of flow angle between the horizontal plane and approximately 45°.

## Patentansprüche

1. Vorrichtung zur Trennung von Blutkomponenten mittels der durch die Schwerkraft verursachten Sedimentationswirkung, enthaltend:

einen ersten Hohlraum (2), der mit einer Öffnung für eine Zuführungsleitung (7) für Blut ausgestattet ist;

eine Komponenten -Trennzone, die mit diesem Hohlraum verbunden ist, wobei das Innere der Trennzone mindestens einen Blut-Strömungskanal (4) bildet, der entweder rohrförmig ist und einen Innenhohlraum mit einem Querschnitt von nicht mehr als 3 cm$^2$ hat oder der eine planare obere Fläche und untere Fläche aufweist, die einen Hohlraum einer Dicke von nicht mehr als 20 mm einschließen; und einen mit der Komponenten-Trennzone verbundenen zweiten Hohlraum (5), der mit mindestens zwei Öffnungen (8, 9) für Entnahmeleitungen für die abgetrennten Blutkomponenten versehen ist.

2. Vorrichtung gemäß Anspruch 1, in der zwei Öffnungen für die Entnahmeleitungen (8, 9) an zwei Stellen unterschiedlicher Höhe in dem zweiten Hohlraum angeordnet sind.

3. Vorrichtung gemäß Anspruch 1, in der drei Öffnungen für Entnahmeleitungen (8, 9, 10) an Stellen unterschiedlicher Höhe in dem zweiten Hohlraum angeordnet sind.

4. Vorrichtung gemäß einem der Ansprüche 1 bis 3, in der ein Damm (29) in mittlerer Höhe in dem zweiten Hohlraum (5) derart angebracht ist, daß der Damm einen Teil des Blutstroms absperrt und in der eine Öffnung (11a) für eine Entnahmeleitung (11) in mittlerer Höhe auf der stromaufwärts von dem Damm (29) liegenden Seite angeordnet ist und mindestens eine Öffnung (30a) für eine Entnahmeleitung auf der Seite stromabwärts von dem Damm angeordnet ist.

5. Vorrichtung gemäß einem der Ansprüche 1 bis 4, in der die Blut-Strömungskanäle (4) in Richtung der Strömung nach oben unter einem Winkel geneigt sind, der zwischen der horizontalen Ebene und etwa 45° liegt.

## Revendications

1. Appareil pour la séparation des composants du sang au moyen d'une action de sédimentation due à la force de la gravitation, comprenant:

un premier espace vide (2) pourvu d'une ouverture pour une ligne d'alimentation (7) du sang;

une portion de séparation des composants connectée audit espace vide, l'intérieur de la portion de séparation formant au moins un canal (4) d'écoulement du sang, qui est soit tubulaire et a un espace interne vide d'une aire en coupe ne dépassant pas 3 cm$^2$ ou a des surfaces supérieure et inférieure planes incorporant un espace vide ayant une épaisseur ne dépassant pas 20 mm; et

un second espace vide (5) connecté à ladite portion de séparation des composants et pourvu d'au moins deux ouvertures (8, 9) pour des lignes d'évacuation des composants séparés du sang.

2. Appareil selon la revendication 1 où deux ouvertures pour les lignes d'évacuation (8, 9) sont placées à deux hauteurs différentes dans le second espace vide.

3. Appareil selon la revendication 1 où trois ouvertures pour les lignes d'évacuation (8, 9, 10) sont placées à des hauteurs différentes dans le second espace vide.

4. Appareil selon l'une quelconque des revendications 1 à 3 où un barrage (29) est monté à une hauteur intermédiaire dans le second espace vide (5) de manière que le barrage bloque une portion de l'écoulement du sang et où une ouverture (11a) pour la ligne d'évacuation (11) est placée à une hauteur intermédiaire du côté amont du barrage (29) et au moins une ouverture (30a) pour une ligne d'évacuation est placée du côté aval du barrage.

5. Appareil selon l'une quelconque des revendications 1 à 4 où les canaux d'écoulement du sang (4) sont inclinés vers le haut dans la direction de l'écoulement à un angle compris entre le plan horizontal et environ 45°.

*Fig. 1*

*Fig. 2*

0 057 907

## Fig. 3

## Fig. 4

## Fig. 5

## Fig. 6

## Fig. 7

## Fig. 8

## Fig. 9

## Fig. 10

## Fig. 11

*Fig.12*

*Fig.13*